# EUROPEAN PATENT SPECIFICATION

(11) **EP 0 977 514 B1**
(45) Date of publication and mention of the grant of the patent: **18.01.2006**
(21) Application number: 97940020.7
(22) Date of filing: 24.07.1997
(51) Int. Cl.: A61B 17/60, A61B 17/64, A61B 19/00

(54) **ORTHOPAEDIC SYSTEM ALLOWING ALIGNMENT OF BONES OR FRACTURE REDUCTION**
ORTHOPÄDISCHE ANORDNUNG ZUR AUSRICHTUNG VON KNOCHEN ODER ZUR BEHANDLUNG VON KNOCHENBRÜCHEN
SYSTEME ORTHOPEDIQUE PERMETTANT D'ALIGNER DES OS OU DE REDUIRE DES FRACTURES

(30) Priority: 25.07.1996 IT CS960011; 27.01.1997 IT CS970004
(43) Date of publication of application: 09.02.2000
(73) Proprietor: Calabrian High Tech S.r.l., 87036 Rende (CS) (IT)
(72) Inventor: De Luise, Renata, 20122 Milano (IT); Danieli, Guido A., 87030 Arcavacata di Rende (IT)
(86) International application number: PCT/EP1997/004019
(87) International publication number: WO 1998/004203

(56) References cited:
- WO-A-96/19944

## Description

During orthopaedic procedures it is often necessary to reach with a certain degree of precision a particular part of the bone structure either to fix internally a.fracture, or to reach a tumour, or else to align fractured bones before placing an external fixator.

### Background Art

Traditional surgery often operates by trial and error controlling under fluoroscopy the effect of the motion imposed. Sometimes, due to the impossibility of drilling holes knowing the drill position in a given frame of reference, most invasive surgery procedures are used, thereby requiring long periods of recovery for the patient. And, in any event, a massive dose of radiation is received by patient and doctors.

### Disclosure of Invention

The present invention, coupling carefully chosen geometry, kinematic, measurement theory and computer science, provides a new generation of instruments allowing to position objects such as screws and bones, in the desired position, without any absorption of radiation by the doctor, and strongly reducing also the radiation to the patient, that has to receive only two fluoroscopies, used to calibrate the system.

This patent derives from two recent Italian patent applications, CS96A000011, published on October 23, 1996 (ITCS 96 0011) and CS97A000004, published on July 27, 1997 (ITCS 97 0004), and from the experience gained by the author during a surgical procedure to hit on the first attempt a osteoma-osteoide placed in the medial upper portion of the femur of a young lady. A Basic instrument allowing the actual to virtual reality connection, is the goniometer (measuring device) part of WO-A-96/19944 which discloses a system according to the preamble of claim 1.

### System Description

The complete system is composed by four elements, the aiming device (1), the image ca- librating device (2), the regulating device (3) and the measuring device (4) coupled to a computer, also containing an image acquisition board, and specialised software.

The aiming device (1) can be built in many different configurations, as described later, and could be used even only coupled to the regulating device (3), but in this case needs a greater usage of fluoroscopy.

Using the complete system, one can centre screws and similia into a particular portion of the body, working under Virtual Reality conditions. In fact, once the calibration images are acquired and repere point located, the computer will display on its screen in one plane at the time, or in two planes at the same time, the effect of the motion of the distal portion of the instrument with respect to the proximal portion, in the exact scale, thereby allowing the correct alignment, being the doctor helped also by a wise choice of the various degrees of freedom of the regulating device (3).

Vice versa, without the aiming device (1), the system can be used to align bones connected to clamps, in order to obtain a fracture reduction, once again working under Virtual Reality and acting on the clamps of a fixator. Also in this case the doctor will be able to observe the effect of its movements in the exact scale on the computer screen in one or two planes. A possibility of obtaining images also on a third plane is foreseen, and will be obtained via software. Let us now describe in greater details the various components.

### Aiming Device

The aiming device (1) is basically composed by an L shaped structure, whose angle between arms (5) can be fixed or adjustable, being each arm of fixed or adjustable length, each arm bearing two bars (7) lying on the same plane of the arm's hinge axis (6).

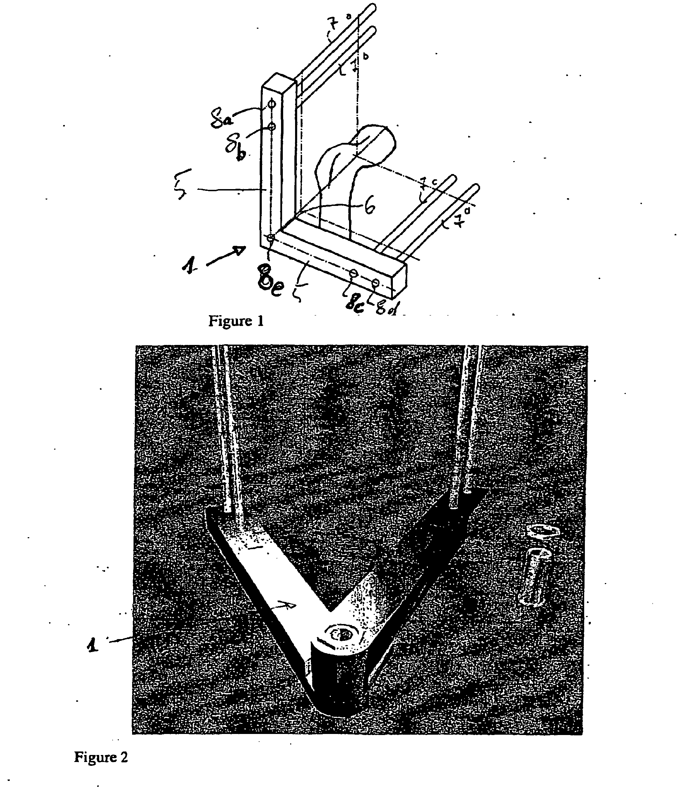

With reference to Figure 1, the simplest possible version of the aligner is made by a one piece element, bearing five holes (8a, 8b, 8c, 8d, 8e) whose axes are parallel, three aligned on a first plane, and the other two also aligned with one of the first three axes. Four aiming bars (7a, 7b, 7c, 7d) are inserted in four centring holes, (8a, 8b, 8c, 8d) while the fifth (8e), aligned with each of the two remaining pairs, is left empty to serve as a guide for the instrument used to reach the region of clinical interest.

A more sophisticated version is depicted in Figure 2, where the angle and the bar's length are adjustable.

Sometimes the centring holes must be more than one, as in the case of fracture of the neck of a femur, where two screws have to be placed in a very little space.

### Image Calibrating Device

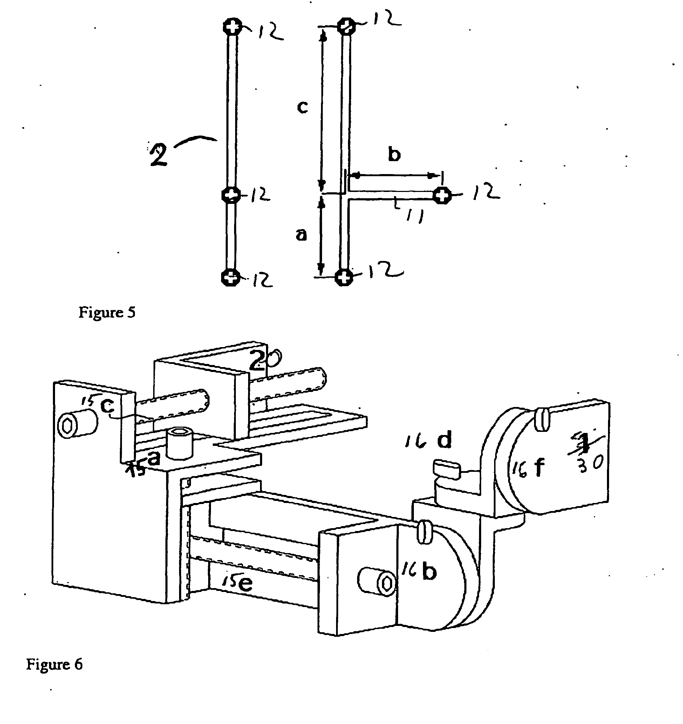

The Image Calibrating Device (2) depicted in figure 5 is essentially made by two light structures (10, 11) that should carry a number of repere points (12) (at least three per side and per image) used to mathematically determine, knowing their physical dimension (a, b, c), and their projection on the two calibrating images acquired by the computer, the picture scale and the angles determining the actual position of the measuring device.

### Regulating Device

The Regulating Device (3) is a mechanism allowing to adjust the position of the movable end (distal) of the instrument (i.e. the aiming device) or fixator, with respect to the fixed (proximal) end, by operating in sequence on independent planes. For this purpose it is composed by a series of hinges and telescopic kinematic pairs which have to be regulated in sequence in order to reach the required alignment. The regulating device may be part of the fixator's body, as in the case of US patent 5,152,280, may be part of the measuring device, or may be independent from both, but must allow independent regulation of the six degrees of freedom between end components working in sequence on independent planes.

In the example of figure 6 is shown a mechanical regulating device acting on independent planes, composed of three telescopic pairs (15a, 15b, 15c) and three hinges (16b, 16d, 16f). In the example of figure 7 is shown a mechanical regulating device acting on independent planes, composed of two telescopic pairs (15c, 15e) and four hinges (16a, 16b, 16d, 16f). In figure 8, is shown a mechanical regulating device acting on independent planes, allowing pure rotation about the diaphysis.

### Measuring Device

The Measuring Device is any device enabling to measure in real time the relative position between two bodies, passing the information directly to a computer. A possible execution of such a device can be the goniometer already quoted, but this is just an example.

### Instrument Alignment Procedure

The alignment procedure under fluoroscopy requires the first two bars (7a, 7b) of the aiming device (1) to be aligned on the region of interest, so that the two bars (7a, 7b) are superimposed to the image of such region, being only one bar in sight. To do this, the doctor will have to act on the regulating device (3) till the first alignment is reached. Next the system is blocked in such a way that it can only move within the first aiming plane. The X-ray apparatus is next rotated, and the second two bars (7c, 7d) are moved to align the region of clinical interest on the second direction. Blocking the device at this point will cause any instrument entering the guiding holes with a reduced tolerance to hit the region of interest.

If virtual reality operation is desired, the alignment on the first plane needs only to be approximate, but the image calibrating device (2) is to be used. On the bars three spheres (or other aiming tool) of known position along the bars have to be located. The first X-ray is performed and the image (displaying the actual bar position with respect to the region of interest) supplied to the computer. Similarly one proceeds with a second X-ray on a plane approximately perpendicular to the first one, carrying also in this case the second pair of bars the minimum three aiming tools.

Once this is done, the computer will first calculate angles and scale on the base of the projections of the image calibrating device (2). Similarly to the fluoroscopic operation, the doctor will then move the distal portion of the instrument acting on the regulating device (3), observing the image as mathematically computed displayed on the computer screen. Once the first plane is centred, the doctor will block the system on a first plane, then proceed on the second plane adjustment. Only difference is that the image is now in Virtual Reality, but reproducing the actual geometry as calibrated by the computer.

Once also this alignment is reached, the introduction of an instrument through the centring hole (8e) will allow, placing the instrument/drill, where it is supposed to go. This time however there is also the possibility, if the geometry of all the tools involved is known, to compute also the penetration, so that the tip of the instrument will be brought to the appropriate depth.

### Bone's Alignment procedure

If alignment of bones to be fixed with external fixator is desired, only the presence of the aiming device is not required, everything else being needed.

To this end, either the fixator itself has a central body allowing to operate in sequence on its internal hinges or telescopes, working in sequence on perpendicular planes, as in the case of the fixator US patent 5,152,280, or a separate regulating device must be used. To this the image calibrating and measuring devices (and relative computer) are to be added, coupling them to the end clamps. The measuring device, such as the one described in WO96/19944 of july 4th 1996, allows computing the effect of the motion performed by the doctor, showing in real time and with millimetric precision on the computer screen the new position of the bone segments in the proper scale.

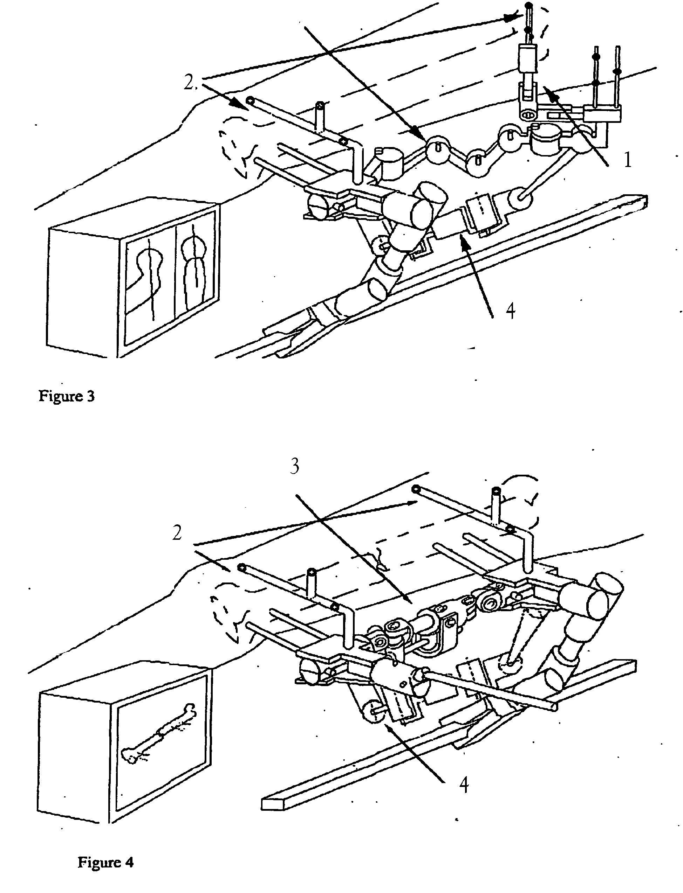

Also in this case it is necessary to supply the computer two images in approximately perpendicular planes. Each image will show, the initial position of the bone segments, but also the position of the six point of known co-ordinates with respect to the measuring device clamps, belonging to the image calibrating device. Once identified their position, either automatically or manually (with the mouse) and acquired their position on the picture, the image calibration is automatically performed. Next the identification of the bones segments, again manual or automatic, follows. Figure 3 presents the system during alignment of instruments, it depict a schematic representation of guiding wires in a particular position. It depicts a schematic representation of guiding wires in a particular position acting under virtual reality. While the following Figure 4 presents a fracture reduction. Figure 5 shows a possible configuration of one image calibrating device (2), of which four are needed (or two rateable), one per bone segment and per reduction plane (frontal and sagittal).

Should the fixator be not provided itself with the regulating device (3), this will have to be provided externally, being manual or electronically actuated.

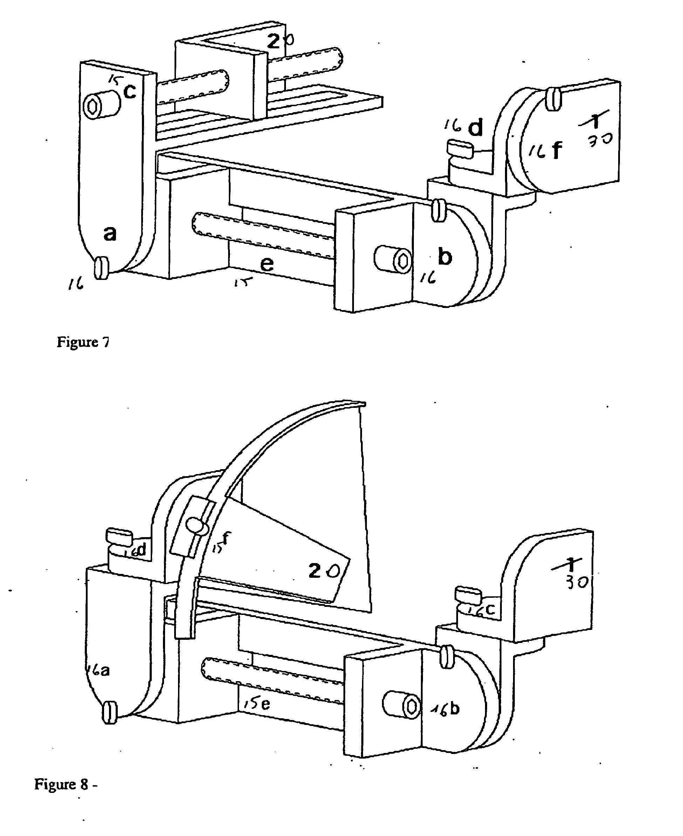

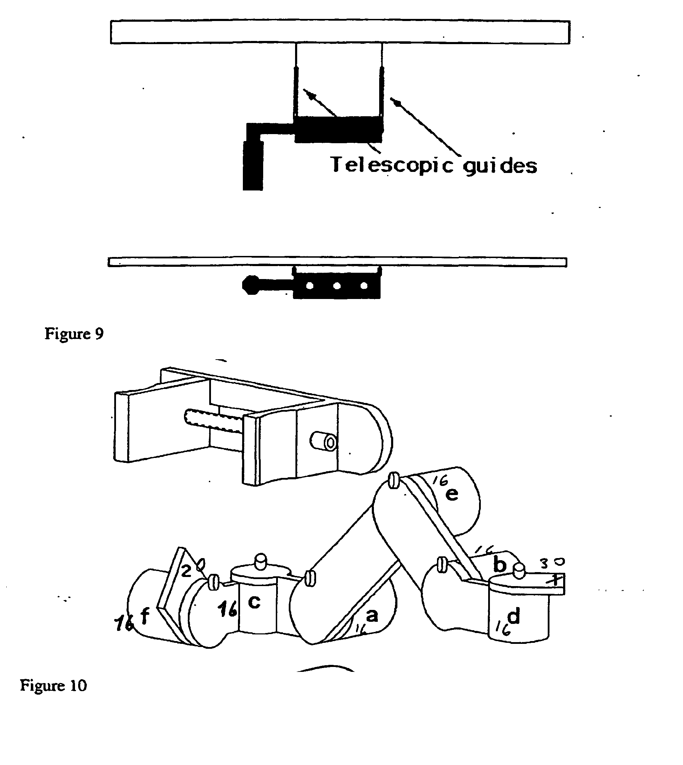

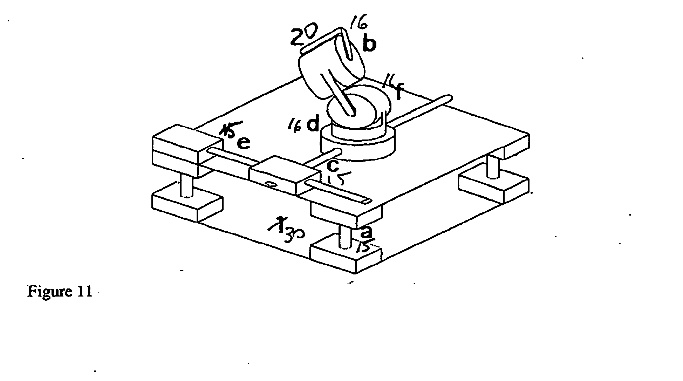

Naturally this device may have a hinge sequence such as the quoted US patent, but may be totally different. In particular a configuration is here proposed in which the six degrees of freedom are supplied by three telescopic pairs (15a, 15b, 15c) and three hinges (16b, 16d, 16f), as shown in Figure 6. Naturally the end clamps have to be connected between the members designated in the Figure as member (20) and (30) respectively. Also notice that the relative position between the various constraints can be varied if necessary. Another possibility is presented in the following Figure 7. To further simplify the operator's work, kinematic pair (15f) can also be made as presented in Figure 8, so that (15e) and (15f) together will allow a pure translation and rotation about the diafisis. Naturally to do so the ring belonging to kinematic pair (15f) must be centred in the diafisis and perpendicular to it. Small errors can however be corrected acting on the remaining constraints (16a, 16b, 16c, 16d) to obtain the correct position of (15f) it is possible to utilise the instrument shown in Figure 9 guiding the insertion of the fixator's screws into the distal portion of the bone so that ring (15f) be perpendicular to the diafisis.

The regulating device can also be coupled to the measuring device, as shown in Figure 10, where (16a, 16b, 16c, 16d, 16e, 16f) are designated as "measuring hinges", and where a possible "extensor device" is shown to be positioned between hinges (16a) and (16b), in order to ease distraction of end clamps.

Last Figure shows a further scheme where the regulating/measuring device is motorised, being operated by simple switches,

As a final point, note that during fracture reduction it is not necessary to mount the regulating device before taking the calibration X-rays, but it is enough if the measuring and image calibrating devices are present. The regulating device can be installed only after the X-rays, thereby having the advantage of being able to have full sight (in V.R.) of the fracture region also from the sagittal standpoint, which is an advantage with respect to traditional fixator assisted reduction procedure.

## Claims

1. Alignment System for instruments or fractures in Virtual Reality, comprising and to be positioned between the end clamps of the instrument: a measuring device (4) bearing six degrees of freedom connected to a computer; a regulating device (3) allowing adjustment of the relative clamp position acting in sequence on independent planes with six degrees of freedom, **characterised in that** if further comprises an image calibrating device (2) allowing identification/calibration subsystem of the relative position between bone and measuring device; an aiming device (1) enabling to centre a given portion of the body and further comprising a video image acquisition board and related specialised software.

2. Alignment System for instruments or fractures in Virtual Reality according to claim 1, **characterised by** the fact that the aiming device (1) is composed by an L shaped structure, whose angle between arms (5) can be fixed or adjustable, being each arm of fixed or adjustable length, each arm (5) bearing two bars (7) lying on the same plane of the arm's hinge axis (6), said L shaped structure, bearing five holes (8a, 8b, 8c, 8d, 8e) whose axes are parallel, three aligned on a first plane, and the other two also aligned with one of the first three axes, wherein in four centring holes (8a, 8b, 8c , 8d) are inserted four aiming bars (7a,7b, 7c, 7d), while the fifth hole (8e), aligned with each of the two remaining pairs, is left empty to serve as a guide for the instrument used to reach the region of clinical interest

3. Alignment System for instruments or fractures in Virtual Reality according to claim 1, **characterised by** the fact that the image calibrating device (2) is made by two light structures that carry at least three repere points (12) per side and per image used to mathematically determine, knowing their physical dimension, and their projection on the two calibrating images acquired by the computer, the picture scale and the angles determining the actual position of the measuring device.

4. Alignment System for instruments or fractures in Virtual Reality according to claim 1, **characterised by** the fact that the regulating device (3) is composed of three telescopic pairs (15c, 15a, 15b) and three hinges (16b, 16d, 16f)

5. Alignment System for instruments or fractures in Virtual Reality according to claim 1, **characterised by** the fact that the measuring device (4) is a device enabling to measure in real time the relative position between two bodies, passing the information directly to a computer.

## Patentansprüche

1. Angleichsystem für Instrumente oder Frakturen in Virtueller Realität, das enthalt und zwischen den Endklammem des Instruments in Position gebracht werden soll, **dadurch gekennzeichnet auch,** dass eine Messvorrichtung (4) mit 4 Grad Spielraum an einen Computer angeschlossenen; das enthalt weiterhin eine Reguliervorrichtung (3) zur Einstellung der jeweiligen Klammerposition durch sequenzielle Einwirkung auf unabhängige Ebenen mit sechs Grad Spielraum, das enthalt weiterhin eine Bildkalibrienungsvorrichtung (2) zur Identifizierung/Kalibrierung der jeweiligen Position zwischen Knochen und Messvorrichtung; das enthalt weiterhin eine Zielvorrichtung (1) zur Zentrierung eines vorgegebenen Körperbereichs, und das enthalt weiterhin einer Videoaufnahmeeinheit mit spezieller Software.

2. Angleichsystem für Instrumente oder Frakturen in Virtueller Realität gemäß anspruch 1, **dadurch gekennzeichnet, dass** die Zielvorrichtung (1) aus einer L-förmigen Struktur besteht, deren Winkel zwischen den Armen (5) fixiert oder variiert werden kann, da jeder Arm eine feste oder variable Länge besitzt, jeder Arm (5) enthält zwei Streben (7), die sich auf der gleichen Ebene der Scharnierachse des Arms (6) befinden, L-förmige Struktur genannt, mit fünf Löchern (8a, 8b, 8c, 8d, 8e), die parallele Achsen besitzen - drei auf der ersten Ebene und die anderen beiden angeglichen an eine der ersten drei Achsen, wobei in die vier Zentrierlöcher (8a, 8b, 8c, 8d) vier Zielstreben (7a, 7b, 7c, 7d) eingefügt sind, während das fünfte Loch (8e), das an jedes der verbleibenden zwei Paare angeglichen ist, leer bleibt, um als Führung für das Instrument zu dienen, das zum Erreichen des klinischen Interessensbereichs verwendet wird.

3. Angleichsystem für Instrumente oder Frakturen in Virtueller Realität gemäß Anspruch 1, **dadurch gekennzeichnet, dass** die Bildkalibrierungsvorrichtung (2) aus zwei leichten Strukturen besteht, die mindestens drei Orientierungspunkte (12) pro Seite und Bild besitzen, um die Bildskala und die Winkel mathematisch zu bestimmen, die, unter Kenntnis der physikalischen Größe und der Projektion auf den beiden durch den Computer erhaltenen Kalibrierungsaufnahmen, die tatsächliche Position der Messvorrichtung festlegen.

4. Angleichsystem für Instrumente oder Frakturen in Virtueller Realität gemäß Anspruch 1, **dadurch gekennzeichnet, dass** die Reguliervorrichtung (3) aus drei Teleskoppaaren (15c, 15a, 15b) und drei Scharnieren (16b, 16d, 16f) besteht.

5. Angleichsystem für Instrumente oder Frakturen in Virtueller Realität gemäß Anspruch 1, **dadurch gekennzeichnet, dass** die Messvorrichtung (4) eine Vorrichtung zur Echtzeitmessung der jeweiligen Position zwischen zwei Körpern ist, die die Information direkt an den Computer sendet.

## Revendications

1. Système d'alignement pour instruments et ruptures d'os en Réalité Virtuelle, qui comprends et qui doive être positionnée entre les brides extrêmes de l'instrument: **caractérisé par le fait qu'**il comprend aussi un instrument de mesure (4) avec six degrés de liberté couplé a un ordinateur; et comprenant aussi un instrument de régulation (3) de la position relative entre les brides agissant en séquence sur des planes indépendant avec les six degrés de liberté; et comprenant aussi un équipement de calibration des images (2) qui permets de identifier et calibrer la position relative entre les os et l'instrument de mesure; et comprenant aussi un appareil pour viser (1) qui permet de centrer une partie donnée du corps; et comprenant aussi un circuit électronique d'acquisition vidéo et le relatif software spécialisé.

2. Système d'alignement pour instruments et ruptures d'os en Réalité Virtuelle selon la première revendication, **caractérisé par le fait que** l'appareil pour viser (1) est formé par une structure à L, dont l'angle entre les bras (5) peut être réglable ou fixé, étant chaque bras de longueur fixé ou réglable, chaque bras soutenant deux barres (7) qui se trouvent sur le même plan de l'axe de la charnière des bras (6), ladit structure à L, soutenante cinq trous (8a, 8b, 8c, 8d, 8e) dont les axes sont parallèles, trois alignés sur un premier plan, et les autres deux également alignés avec un des trois premières axes, où en quatre trous de centrage (8a, 8b, 8c, 8d) sont insérés quatre barres visantes (7a, 7b, 7c, 7d), alors que le cinquième trou (8e), aligné avec chacune des deux paires restantes, est laissé vide pour servir de guide à l'instrument utilisé pour atteindre la région d'intérêt clinique.

3. Système d'alignement pour des instruments ou des ruptures d'os en réalité virtuelle selon la revendication 1, **caractérisé par le fait que** le dispositif de calibrage d'image (2) est fait par deux structures légères qui portent au moins trois points de repere (12) par côté et par image employée pour déterminer mathématiquement, sachant leur dimension physique, et leur projection sur les deux images de calibrage acquis par l'ordinateur, la balance d'image et les angles déterminant la position actuel de l'appareil de mesure.

4. Système d'alignement pour des instruments ou des ruptures d'os en réalité virtuelle selon la revendication 1, **caractérisé par le fait que** le dispositif de régulation (3) se compose de trois paires télescopiques (15c, 1ä, 15b) et de trois charnières (16b. 16d, 16f).

5. Système d'alignement pour des instruments ou des ruptures d'os en réalité virtuelle selon la revendication 1, **caractérisé par le fait que** l'appareil de mesure (4) est un dispositif permettant: de mesurer en temps réel la position relative entre deux corps, passant l'information directement à un ordinateur.
